# EUROPEAN PATENT SPECIFICATION

(11) **EP 2 626 704 B1**
(45) Date of publication and mention of the grant of the patent: **18.11.2015**
(21) Application number: 12154587.5
(22) Date of filing: 08.02.2012
(51) Int. Cl.: G01N 33/68

(54) **DIAGNOSIS OF PANCREATIC DIABETES IN PATIENTS WITH NORMAL BLOOD LEUCOCYTE COUNTS USING PROCALCITONIN**
DIAGNOSE VON BAUCHSPEICHELDRÜSEN DIABETES BEI PATIENTEN MIT NORMALEN BLUT LEUKOZYTENZAHLEN UNTER VERWENDUNG VON PROCALCITONIN
DIAGNOSTIC DU DIABÈTE PANCRÉATIQUE CHEZ LES PATIENTS AVEC LES VALEURS NORMALES DE LEUCOCYTES SANGUINS EN UTILISANT LA PROCALCITONINE

(43) Date of publication of application: 14.08.2013
(73) Proprietor: Soylemez, Mehmet Ali, 34668 Istanbul (TR)
(72) Inventor: Soylemez, Mehmet Ali, 34668 Istanbul (TR)

(56) References cited:
- US-A1- 2011 263 438
- M.A. STOYLEMEZ: "Procalcitonin is a specific marker of diabetic complication process", EUROPEAN JOURNAL OF HUMAN GENETICS, vol. 18, no. suppl 1, 12 June 2010 (2010-06-12), page 352, XP055037335, Gothenburg, Sweden
- SOYLEMEZ ET AL: "A Novel Mechanism between Diabetes Mellitus Complications and Procalcitonin Gene Expression", MOLECULAR THERAPY, ACADEMIC PRESS, SAN DIEGO, CA, USA, vol. 13, 1 January 2006 (2006-01-01), page S86, XP005675332, American Society of Gene & Cell Therapy 555 East Wells Street Suite 1100 Milwaukee, WI 53202 USA ISSN: 1525-0016
- SOYLEMEZ ET AL: "894. A Novel Mechanism between Type II Diabetes Mellitus and Procalcitonin Gene Expression", MOLECULAR THERAPY, ACADEMIC PRESS, SAN DIEGO, CA, USA, vol. 11, 15 August 2005 (2005-08-15), page 346, XP005016233, American Society of Gene & Cell Therapy 555 East Wells Street Suite 1100 Milwaukee, WI 53202 USA ISSN: 1525-0016
- ABBASI A ET AL: "Plasma procalcitonin and risk of type 2 diabetes in the general population", DIABETOLOGIA ; CLINICAL AND EXPERIMENTAL DIABETES AND METABOLISM, SPRINGER, BERLIN, DE, vol. 54, no. 9, 15 June 2011 (2011-06-15), pages 2463-2465, XP019934333, ISSN: 1432-0428, DOI: 10.1007/S00125-011-2216-3
- MESUT MUTLUOLU ET AL: "Can procalcitonin predict bone infection in people with diabetes with infected foot ulcers? A pilot study", DIABETES RESEARCH AND CLINICAL PRACTICE, AMSTERDAM, NL, vol. 94, no. 1, 16 May 2011 (2011-05-16), pages 53-56, XP028323696, ISSN: 0168-8227, DOI: 10.1016/J.DIABRES.2011.05.023 [retrieved on 2011-05-19]

## Description

The Invention relates to a method for the diagnosis of pancreatic ***diabetes*** mellitus and/or diabetic foot syndrome, wherein a determination of the marker procalcitonin (PCT: SEQ ID No. 1) is carried out on a patient with blood leucocyte counts within normal value ranges. The diagnostic test must be used only on a patient within normal value ranges of leucocytes. Otherwise, the test should not be used for diagnosis of pancreatic diabetes mellitus and/or diabetic foot syndrome.

The diagnosis of pancreatic ***diabetes*** mellitus and/or diabetic foot syndrome by procalcitonin has been disclosed in the prior art (Mehmet Ali Soylemez et al. A Novel Mechanism between Type II Diabetes Mellitus and Procalcitonin Gene Expression Molecular Therapy (2005) 11, S346|[ndash]|S346; doi: 10.1016/j.ymthe.2005.07.437 and Mehmet A. Soylemez et al. A Novel Mechanism between Diabetes Mellitus Complications and Procalcitonin Gene Expression. Molecular Therapy (2006) 13, S86|[ndash]|S86; doi: 10.1016/j.ymthe.2006.08.250). However, the present invention discloses for the first time that the above is only valid for patients with normal value ranges of blood leucocytes.

The use of procalcitonin (PCT) determinations in diagnosis is described in the prior art, particularly with regard to an investigation of sepsis (Yukioka et al., Ann. Acad. Med. Singapore 30: 528-31 (2001)). However, the suitability of procalcitonin (PCT: SEQ ID No. 1) for the diagnosis and complications of pancreatic ***diabetes*** mellitus are only disclosed in the above recited publications by soylemez et al.

The present invention discloses an improved method for the diagnose of pancreatic ***diabetes*** mellitus and/or diabetic foot syndrome in patients with normal blood leucocyte counts.

This task is accomplished by means of a method according to the appended claims.

Although not in accordance with the invention as claimed, the method could also be used for "risk stratification". The term "risk stratification," according to the invention, comprises finding ***diabetes*** patients, particularly those having diabetic sequelae, with the worse prognosis, for the purpose of intensive diagnosis and therapy/treatment (of sequelac) of pancreatic ***diabetes*** mellitus, with the goal of allowing as advantageous a course of the pancreatic ***diabetes*** mellitus as possible.

For this reason, it is particularly advantageous that a reliable diagnosis and/or risk stratification can take place by means of the method according to the invention. The method according to the invention allows clinical decisions that lead to the treatment or therapy of pancreatic ***diabetes*** mellitus and complications. Invention is related to possible new drug development for the treatment of pancreatic diabetes mellitus and/or complications.

In serum, procalcitonin (PCT) has a half-life of 25 to 30 hours (Karzai et al. Procalcitonin-A New Indicator of the Systemic Response to Severe Infections. INFECTION Volume 25, Number 6, 329-334) Therefore, PCT test results could give enough knowledge for the evaluation of diabetic complication damage in a I- day period. So, it is possible to prevent daily diabetic complications' damage by determining the right drug and diet therapy and/or invent new drugs and explore of new diet therapies for pancreatic diabetes patients.

Although not in accordance with the invention as claimed, in vitro method for prognosis for a patient having diabetic and/or prediabetic stage disease could be possible, the method comprising: determining the level of procalcitonin in a sample selected from the group consisting of a blood sample, a serum sample and a plasma sample obtained from the patient with blood leucocyte counts within normal value ranges and comparing the level of procalcitonin to a threshold level, whereby, when the level of procalcitonin exceeds said threshold level, the patient is diagnosed with pancreatic diabetes mellitus and/or diabetic complications, and wherein said threshold level is 0.03±0.002ng/ml (Please see: Table 2, Figure-2; Table-1, Figure-1).

Normal value ranges of procalcitonin threshold level at pancreatic diabetes mellitus diagnosis and/or complications may vary slightly among different laboratories.

In another preferred embodiment of the method diagnosis and/or risk stratification take place, for detection and detection by means of differential diagnosis, for assessment of the degree of severity, and for assessment of the course of pancreatic ***diabetes*** mellitus and complications as an accompaniment to the therapy.

In another preferred embodiment of the method, samples of body fluids, particularly blood, optionally whole blood, serum, or available plasma, are taken from the patient to be investigated, and the diagnosis takes place in vitro/ex vivo, i.e. outside of the human or animal body. The diagnosis and/or risk stratification can take place on the basis of the determination of the marker procalcitonin (PCT: SEQ ID No. 1), and its amount that is present, or a change in amount, as compared with a reference, in at least one patient sample within normal value ranges of leucocytes.

Within this invention, the term "pancreatic ***diabetes*** mellitus," particularly Type II diabetes mellitus (insulin-resistant), is understood to mean a chronic inflammatory metabolic disease, where the production of insulin in the beta cells of the islets of Langerhans in the pancreas is disturbed, or insulin is present but cannot correctly act at its target location, the cell membranes. The results of this disturbed insulin production and effect are elevated blood sugar values (hyperglycemia). In the diabetic disease profile, a differentiation is made between prediabetes, in which a "disturbed glucose tolerance," which can be detected by laboratory chemistry, occurs only in the end stage, and actual manifest pancreatic ***diabetes*** mellitus. Insulin resistance stands at the beginning of the prediabetic illness phase. Almost at the same time, endothelial dysfunction already develops, along with hyperlipoproteinemia and hypertensive dysfunction of the cardiovascular system. The results of this risk constellation are furthermore artherosclerotic changes in the blood vessel walls (microangiopathy and macroangiopathy), as well as vascular complications as the result of microcirculation problems. Other sequelae and concomitant illnesses are diabetic retinopathy going as far as blindness, as well as nephropathy going as far as renal insufficiency, neuropathy, the diabetic foot syndrome, and cardiovascular complications. Diabetic complications is characterized by a chronic process, which commences years before pancreatic diabetes mellitus becomes overt. Each step of pathogenetic cascade is accompanied by inflammation (Otter W., Standl E., Schnell O, Inflammation and atherogenesis in diabetes mellitus new therapeutic approaches. Herz.29(5);524- 31, 2004). Procalcitonin is elevated by inflammatory processes, with greater specificity and sensitivity than other acute phase proteins (Hatheril M., Tibby SM., Sykes K., Turner C., Murdoch, IA., Diagnostic markers of infection: comparision of procalcitonin with C reactive protein and leucocyte count. Arch Dis Child). For this reason, the invention relates to the diagnosis and/or risk stratification of Type II ***diabetes*** mellitus within normal value ranges of leucocytes, and its following sequelae and concomitant illnesses: Endothelial dysfunction, hyperlipoproteinemia, hypertensive dysregultrtion of the cardiovascular system, diabetic retinopathy, nephropathy, renal insufficiency, neuropathy, diabetic foot syndrome, and cardiovascular complications.

All the aforementioned indications are furthermore described in Otter W., Standl E., Schnell O., Inflammation and atherogenesis in diabetes mellitus-new therapeutic approaches. Herz. 29(5):524-31,2004.

Within this invention, Procalcitonin (abbreviated as: PCT) was originally described in 1984 as a 116-aminoacid protein with a molecular weight of 14.5 kDa (Le Moullec J.M., et al. The complete sequence of human procalcitonin. FEBS Lett., 167 (1), 93-97 (1984)). The PCT gene, referred to as Calc-1, is located on chromosome 11p15.4 and was sequenced in 1989. The promoter has sites for basal transcription factors but more interestingly, also has sites for NF κβ (Nuclear factor κβ) and AP-1(Activator protein-1), factors induced under inflammatory conditions (Whicher J., Bienvenu J., Monneret G., Procalcitonin as an acute phase marker. Ann Clin Biochem. 38(5):483-93, 2001). Type II diabetes mellitus is associated with oxidative stress and elevation of advanced glycation end products (AGEs). AGEs are produced by a non-enzymatic, Maillard reaction between reducing sugars and either proteins or lipids. AGEs interact the receptor for advanced glycation end products (RAGE) and, RAGE activation is caused by elevation of transcriptional factors NF κβ and AP-1 (Beckman J., Creager M., Libby P., Diabetes and Atherosclerosis JAMA 287: 2570- 2581, 2002). These factors (NF κβ and AP-1) induce procalcitonin gene expression (Mehmet Ali Soylemez et al. A Novel Mechanism between Type II Diabetes Mellitus and Procalcitonin Gene Expression Molecular Therapy (2005) 11, S346|[ndash]|S346; doi: 10.1016/j.ymthe.2005 .07.437). This algorithmic mechanism was based on genetic expression of procalcitonin and first time showed by my study. Results of my study and experimental design imply procalcitonin as an important mediator during the development of diabetic complications and/or pancreatic diabetes mellitus. Thus, the results of this study show that procalcitonin (PCT: SEQ ID No. 1) is a new marker for pancreatic diabetes mellitus and/or diabetic patients with complications (Mehmet Ali Soylemez; 'Procalcitonin is a specific marker of diabetic complication process' (Control No. 2010-A-377) ESHG conference 2010, Gothenburg, Sweden; EJHG volume 18 supl.1, Metabolic disorders Page:352, P13.14). This algorithmic mechanism is strictly related with normal value ranges of leucocytes in human or animal body. If leucocyte count of blood, serum, plasma and/or body fluids in human or animal is above and/or below of normal range, this mechanism will not work.

A leucocyte (leukocyte) count is also known in the prior art as the white blood cell (WBC) . A high WBC count can be an indicator of an infection, inflammation, or allergy. Some conditions may cause a decrease in the WBC count (Leukopenia). Leukopenia may occur as a result of chemotherapy, radiation therapy, viral infections or immune system disorders. Normal value ranges of leucocytes may vary slightly among different laboratories Leucocytes are mainly five types (neutrophil, lymphocyte, monocyte, basophil and eosinophil).

Chronic low grade inflammation is a key feature of the pathophysiology of pancreatic type 2 diabetes. New researches supported this invention and showed plasma procalcitonin to be independent predictor of incident type 2 diabetes mellitus in the general population. Plasma procalcitonin levels are associated with incident type 2 diabetes independent of common diabetes risk factors (Abbasi A et al., "Plasma procalcitonin and risk of type 2 diabetes in the general population", Diabetologia; Clinical and Experimental Diabetes and Metabolism, Springer Berlin, DE, Vol.54, no.9, pages:2463-2465, 15 June 2011). In particular, plasma procalcitonin was more strongly associated with incident type 2 diabetes than other pancreatic diabetic markers. However, infected body parts of patients with pancreatic diabetes cannot support that procalcitonin is an infection marker of diabetic complication process. For instance; bone infection in people with diabetes with infected foot ulcers cannot show the procalcitonin, a novel infection marker, is superior to conventional infection markers in the diagnosis of diabetic foot infection (Mesut Mutluoglu et al: Can procalcitonin predict bone infection in people with diabetes with infected foot ulcers? A pilot study, Diabetes Research and Clinical Practice, Amsterdam, NL, vol.94, no.1, Pages:53-56, 16 may 2011).

Furthermore, the "procalcitonin" according to the invention can demonstrate modifications such as glycolization, lip(o)idiation, or derivatization.

In another embodiment, the determination of procalcitonin (PCT: SEQ ID No. 1) can additionally take place with other markers, where the procalcitonin (PCT: SEQ ID No. 1) is contained in a marker combination (panel, cluster), specifically and preferably with those that already indicate pancreatic ***diabetes*** mellitus. In another particular embodiment, this can also be a vascular marker that can indicate an endothelial dysfunction of the cardiovascular system that accompanies ***diabetes***.

According to the invention, the inflammatory marker can be selected from at least one marker of the group of C-reactive protein (CRP), cytokines, such as TNF-alpha, for example, interleukins, such as IL-6, interleukin-1.beta, proadrenomedullin (proADM), midregional proadrenomedullin (MR-proADM) angiotensin II, endothelin-1, and adhesion molecules, such as VCAM or ICAM, and the vascular marker can be selected from at least one marker of the group of creatine kinase, myeloperoxidase, myoglobin, natriuretic protein, particularly ANP (or ANF), proANP, NT-proANP, BNP, proBNP or NT-proBNP. Furthermore, this term is also understood to mean (pro)hormones that regulate the cardiovascular system, particularly such as pro-gastrin-releasing peptide (proGRP), pro-endothelin-1, pro-leptin, pro-neuropeptide-Y, pro-somatostatin, pro-neuropeptide-YY or pro-opiomelanocortin.

In another embodiment, at least one diabetic marker/factor can additionally be determined. Diabetic markers/factors are, according to the invention, particularly those such as adiponectin, carbohydrates, fats, such as cholestcrols and others, Body Mass Index (BMI), age, blood pressure, HOMA-IR (Homeostasis Model Assessment-insulin Resistance index, for a determination see: Matthews D R, Hosker J P, Rudenski A S, Naylor B A, Treacher D F, Turner R C, Homeostasis Model Assessment: Insulin Resistance and B-cell Function from Fasting Plasma Glucose and Insulin Concentrations in Man. Diabetologia 28:412-419. 1985).

In another embodiment of the Invention, the method according to the invention can be carried out by means of parallel or simultaneous determinations of the markers (e.g. multi-titer plates with 96 cavities and more); where the determinations are carried out on at least one patient sample within normal value ranges of leucocytes.

Furthermore, the method according to the invention and its determinations can be carried out using an automated analysis device, particularly using a Kryptor (http://www.kryptor.net/). Automated analysis device or kryptor works only within normal value ranges of leucocytes. If not, the device gives an error message.

Another task is making available a corresponding diagnostic device, or the use of such a device for carrying out the methods according to the invention.

Within this invention, such a diagnostic device is particularly understood to be an array or assay (e.g. immune assay, ELISA, etc.), in the broadest sense a device for carrying out the method according to the invention.

The following examples and figures serve for a more detailed explanation of the invention, but without restricting the invention to these examples and figures.

### EXAMPLES

### Example 1

Serum procalcitonin levels were analyzed with kryptor analizator. Kryptor-PCT is a kit designed for kryptor automated immunofluorescent assay of procalcitonin in serum.

Statistical analysis was performed by using SPSS method. The results were expressed as mean ± standard deviation. Individuals between group comparisons were made using Student's-T test. Significance was defined at *P*≤ 0.05.

All test subjects of blood leucocytes count was normal and within normal value ranges of leucocytes (Our laboratory normal value ranges of leucocytes: 4000/mm³ - 10000 /mm³).

Procalcitonin was determined in 15 healthy test subjects within normal value ranges of leucocytes with undisturbed glucose tolerance, 18 patients within normal value ranges of leucocytes having manifest diabetes mellitus type 2 without complication (abbreviated as: "DM II") and 15 diabetics who were suffering from diabetic foot complication within normal value ranges of leucocytes.

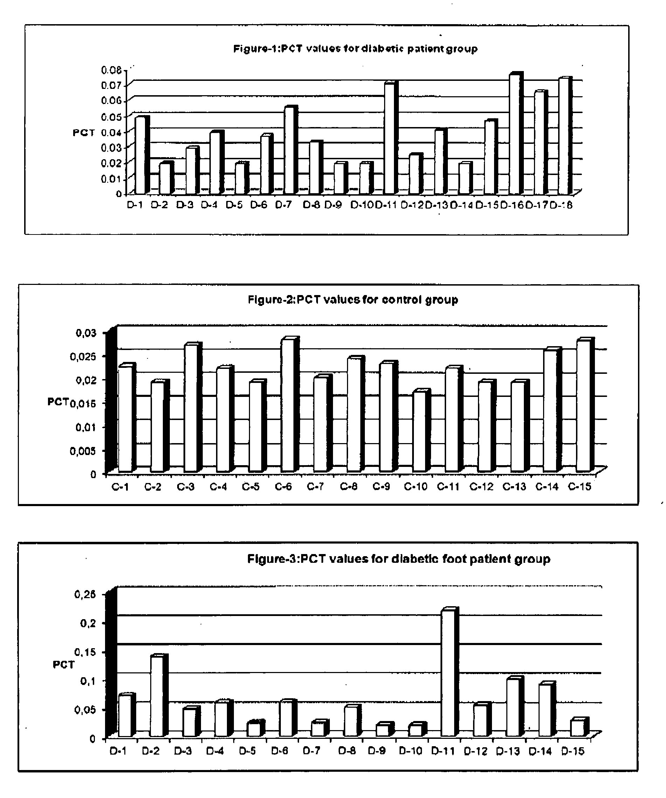

FIG. 2 and Table-2 show procalcitonin in healthy test subjects, patients having type II diabetes mellitus(DM II) without complications (FIG. 1, Table-1), and patients having DM II with diabetic foot complications (FIG. 3, Table-4).

FIGS. 1, 2 and 3 show a significant increase in the procalcitonin values with an increasing degree of severity of DM II. The two groups of DM II patients, in particular, differ from the healthy controls. Surprisingly, the highest procalcitonin values were found in the DM II patients who already demonsrated diabetic foot complication.

Tables-1, 2 and 4 show not only procalcitonin but also the parameters relevant to diabetes, such as glucose, HbAlc and leucocyte in the groups, in each instance.

Procalcitonin (PCT) levels were elevated in type 2 diabetic patients when compared with normal, non-diabetic subjects. There was a statistically significant difference in serum procalcitonin of type 2 diabetic patients versus normal subjects (P<0.01) (see Table. 3).

Procalcitonin level were elevated in diabetic foot complication of patients when compared with normal non-diabetic subjects. There was a statistically significant difference in serum procalcitonin of diabetic foot complication of patients versus normal subjects (P<0.01) (see Table. 5).

### Tables

Table 1 shows not only procalcitonin but also the parameters relevant to diabetes, such as blood sugar (glucose), HbAlc and leucocyte count, in the diabetes patient group, in each instance.

Table 2 shows not only procalcitonin but also glucose, HbAlc and leucocyte values, in the control (non-diabetic) group, in each instance.

Table 3 shows PCT statistical differences of between diabetes patient and control (non-diabetic) groups.

Table 4 shows procalcitonin, glucose, HbAlc and leucocyte values, in the diabetic foot patient group, in each instance.

Table 5 shows PCT statistical differences of between diabetic foot patient and control (non-diabetic) groups.

### FIGURES

FIG.1 shows the results of a graphical representation of diabetes patient group procalcitonin values;
FIG.2 shows the results of a graphical representation of control group procalcitonin values;
FIG.3 shows the results of a graphical representation of diabetic foot patient group procalcitonin values;
Sequence listing shows the results of procalcitonin (abbreviated as: PCT) with the related partial sequences.

Sequence CWU 1

**Table-1: Diabetes patient group parameters: blood sugar, procalcitonin, HbA_{1c} and leucocyte values.**

**Table 1**

| **Patient No:** | **Blood Sugar (mg/dl)** | **Procalcitonin (ng/ml)** | **HbA_{1c (%)}** | **Leucocyte (/mm³)** |
|---|---|---|---|---|
| **D-1** | **179** | **0,0489** | **8.1** | **6440** |
| **D-2** | **248** | **0,0190** | **10.1** | **8480** |
| **D-3** | **223** | **0,0291** | **9.4** | **5370** |
| **D-4** | **123** | **0,0392** | **8.5** | **7890** |
| **D-5** | **157** | **0,0190** | **7.0** | **7920** |
| **D-6** | **158** | **0,0367** | **10.4** | **6330** |
| **D-7** | **172** | **0,0553** | **7.5** | **9140** |
| **D-8** | **304** | **0,0333** | **11.2** | **7770** |
| **D-9** | **138** | **0,0190** | **7.2** | **5130** |
| **D-10** | **370** | **0,0190** | **10.4** | **6440** |
| **D-11** | **168** | **0,0710** | **8.0** | **8070** |
| **D-12** | **155** | **0,0252** | **9.7** | **8880** |
| **D-13** | **355** | **0,0409** | **11.6** | **5770** |
| **D-14** | **170** | **0,0190** | **9.2** | **7700** |
| **D-15** | **95** | **0,0464** | **7.2** | **8400** |
| **D-16** | **107** | **0,0768** | **8.7** | **9400** |
| **D-17** | **289** | **0,0650** | **9.3** | **8560** |
| **D-18** | **144** | **0,0740** | **5.9** | **6800** |
| **Arithmetic average:** | **197.5** | **0.0409** | **8.85** | **7471.6** |
| **SD:** | **82.5** | **0.02** | **1.56** | **1308.40** |

**Table-2: Control group parameters: blood sugar, procalcitonin, HbA1c and leucocyte values.**

**Table 2**

| **Control No** | **Blood Sugar (mg/dl)** | **Procalcitonin (ng/ml)** | **HbA_{1c (%)}** | **Leucocyte (/mm³)** |
|---|---|---|---|---|
| **C-1** | **101** | **0,0224** | **6,1** | **5120** |
| **C-2** | **102** | **0,0190** | **5,5** | **5880** |
| **C-3** | **102** | **0,0269** | **6,2** | **6330** |
| **C-4** | **89** | **0,0220** | **5,6** | **4740** |
| **C-5** | **92** | **0,0190** | **5,8** | **5420** |
| **C-6** | **96** | **0,0281** | **5,4** | **6270** |
| **C-7** | **93** | **0,02** | **5,8** | **5970** |
| **C-8** | **90** | **0,024** | **5,6** | **7890** |
| **C-9** | **94** | **0,023** | **5,8** | **5220** |
| **C-10** | **113** | **0,017** | **6,2** | **9200** |
| **C-11** | **93** | **0,022** | **5,7** | **7300** |
| **C-12** | **90** | **0,0190** | **5,7** | **7270** |
| **C-13** | **91** | **0,00190** | **5,6** | **6300** |
| **C-14** | **97** | **0.0260** | **5,6** | **6390** |
| **C-15** | **80** | **0,028** | **6,1** | **5730** |
| **Arithmetic Average:** | **94,86667** | **0,02236** | **5,78** | **6335,333** |
| **SD:** | **7,595738** | **0,0036** | **0,256905** | **1172,518** |

**Table-3: PCT statistical differences of patient and control groups:**

**Table 3**

| | **Control Group: (Avg ± S.D)** | **Patient Group: (Avg ± S.D)** |
|---|---|---|
| **PCT(ng/ml)** | **0,022 ± 0.0036** | **0,040933 ± 0.02**** |

| | | |
|---|---|---|
| **(**):p<0.01** | | |

**Table-4: Diabetic foot patient group parameters: blood sugar, procalcitonin, HbA_{1c} and leucocyte values.**

**Table 4**

| **Patient No** | **Blood Sugar (mg/dl)** | **Procalcitonin (ng/ml)** | **HbA_{1c (%)}** | **Leucocyte (/mm³)** |
|---|---|---|---|---|
| **DF-1** | **161** | **0,0704** | **13,6** | **9250** |
| **DF-2** | **152** | **0,1378** | **6,3** | **6500** |
| **DF-3** | **227** | **0,0472** | **8,5** | **7300** |
| **DF-4** | **216** | **0,0582** | **9,1** | **6540** |
| **DF-5** | **422** | **0,0227** | **14,2** | **9440** |
| **DF-6** | **100** | **0,059** | **6,5** | **7410** |
| **DF-7** | **100** | **0,0231** | **9,4** | **5610** |
| **DF-8** | **135** | **0,0501** | **7,2** | **7640** |
| **DF-9** | **169** | **0,0190** | **7,9** | **8840** |
| **DF-10** | **148** | **0,0190** | **7,4** | **7570** |
| **DF-11** | **74** | **0,2177** | **6,7** | **8590** |
| **DF-12** | **111** | **0,0544** | **9,6** | **8210** |
| **DF-13** | **69** | **0,0991** | **6,6** | **8720** |
| **DF-14** | **128** | **0,0887** | **8,2** | **8060** |
| **DF-15** | **190** | **0,028** | **11,8** | **9740** |
| **Arithmetic average:** | **160,1333** | **0,066293** | **8,866667** | **7961,333** |
| | | | | |
| **SD:** | **86,34885** | **0,053602** | **2,518408** | **1180,417** |

**Table-5: PCT statistical differences of diabetic foot patient group and control group:**

**Table 5**

| | **Control Group: (Avg ± S.D)** | **Patient Group: (Avg ± S.D)** |
|---|---|---|
| **PCT(ng/ml)** | **0,022 ± 0.0036** | **0,066293 ± 0.05**** |

| | | |
|---|---|---|
| **(**):p<0.01** | | |

Applicant Name: Mehmet Ali SOYLEMEZ
   Title of the Invention: Diagnosis and Complication Risk Assesment of
   Pancreatic Diabetes Within Normal Value Ranges of Leucocytes using
   Procalcitonin
   Date recorded: 07/02/2012
   Operating system used: MS Windows XP
   Reference Number: -
   Application Number:
   Filling Date:

### SEQUENCE LISTING

<110> Soylemez, Mehmet Ali
<120> DIAGNOSIS AND COMPLICATION RISK ASSESSMENT OF PANCREATIC DIABETES
   WITHIN NORMAL VALUE RANGES OF LEUCOCYTES
   USING PROLOCATION
<130> 1
<140> PatentIn version 3.5
<210> 1
   <211> 116
   <212> PRT
   <213> Homo sapiens
<400> 1
   Ala Pro Phe Arg Ser Ala Leu Glu Ser Ser Pro Ala Asp Pro Ala Thr
   1               5                   10                  15
   Leu Ser Glu Asp Glu Ala Arg Leu Leu Leu Ala Ala Leu Val Gln Asp
               20                  25                  30
   Tyr Val Gln Met Lys Ala Ser Glu Leu Glu Gln Glu Gln Glu Arg Glu
           35                  40                  45
   Gly Ser Ser Leu Asp Ser Pro Arg Ser Lys Arg Cys Gly Asn Leu Ser
       50                  55                  60
   Thr Cys Met Leu Gly Thr Tyr Thr Gln Asp Phe Asn Lys Phe His Thr
   65                  70                  75                  80
   Phe Pro Gln Thr Ala Ile Gly Val Gly Ala Pro Gly Lys Lys Arg Asp
                   85                  90                  95
   Met Ser Ser Asp Leu Glu Arg Asp His Arg Pro His Val Ser Met Pro
               100                 105                 110
   Gln Asn Ala Asn
           115

## Claims

1. Procalcitonin immunoassay for in vitro diagnosis of a patient having pancreatic diabetes mellitus and/or diabetic foot syndrome, comprising: determining the level of procalcitonin in a sample selected from the group consisting of a blood sample, a serum sample and a plasma sample obtained from the patient with blood leucocyte counts within normal value ranges of 4000 to 10000 leucocytesper mm³ and comparing the level of procalcitonin to a threshold level, whereby, when the level of procalcitonin exceeds said threshold level, the patient is diagnosed with pancreatic diabetes mellitus and/or diabetic foot syndrome, and wherein said threshold level is 0.03±0.002ng/ml.

2. Method according to claim 1, wherein additionally at least one inflammatory marker is determined, which is selected from the group of C-Reactive protein (CRP), cytokines such as TNF-alpha for example, interleukins, such as IL-6, interleukin-1.beta, proadrenomedullin (proADM), midregional proadrenomedullin (MR-proADM), angiotensin II or endothelin-1.

3. Method according to claim 1, wherein additionally at least one vascular marker is determined, which is selected from the group of creatine kinase, myeloperoxidase, myoglobin, natriuretic protein, particularly ANP (or ANF), proANP, NT-proANP, BNP, proBNP, NT-proBNP, or (pro)hormones that regulate the cardiovascular system, such as pro-gastrin-releasing peptide (proGRP), pro-endothelin-1, pro-leptin, pro-neuropeptide-Y, pro-somatostatin, pro-neuropeptide-YY or pro-opiomelanocortin.

4. Method according to claims 1, 2 and 3 **characterized in that** parallel or simultaneous determinations of the markers are carried out.

5. Method according to claim 1, **characterized in that** the determinations are carried out using an automated analysis device.

## Patentansprüche

1. Procalcitonin Immunassay zur in vitro-Diagnose eines Patienten mit Pankreas-Diabetes mellitus und/oder diabetischen Fuß-Syndrom, einschließlich: Bestimmung des Procaleitonin-Spiegels durch eine Probe ausgewählt aus der Gruppe, in Form einer Blutprohe, eine Serum- und Plasmaprobe eines Patienten mit einer normalen Leukozytenzahl im Wertbereich 4000 bis 10000 Leukozyten per mm3 und Vergleich des Procalcitonin-Spiegels mit einem Schwellenwert, falls der Procalcitonin-Spiegel den besagten Schwellenwert überschreitet wird der Patient mit Pankreas-Diabetes und/oder diabetischem Fuß-Syndrom diagnostiziert wobei der Schwellenwert 0.03±0.002ng/ml ist.

2. Verfahren gemäß Anspruch 1, wobei zusätzlich mindestens ein Entzündungsmarker bestimmt ist, welcher aus einer Gruppe von C-reaktivem Protein (CRP), Cytokine wie zum Beispiel TNF-Alpha, Interleukine wie IL-6, Interleukin-1β, Proadrenomedullin (pro ADM), Midregional-Proadrenomedullin (MR-proADM), Angiotensin II oder Endothelin-1, ausgewählt ist.

3. Verfahren gemäß Anspruch 1, wobei zusätzlich mindestens ein vaskulärer Marker bestimmt ist, welcher aus einer Gruppe von Creatin-Kinase, Myeloperoxidase, Myoglobin, natriuretrisches Protein, besonders ANP (oder ANF), proANP, NT-proANP, BNP, proBNP, NT-proBNP, oder (pro) Hormone, wie zum Beispiel pro-Gastrin-Releasing-Peptide (proGRP), pro-Endothelin-1, pro-Leptin, pro-Neuropeptide-Y, pro-Somatostatin, pro-Neuropeptide-YY oder pro-Opiomelanocortin die das kardiovaskuläre Systems regulieren, ausgewählt ist.

4. Verfahren gemäß Ansprüche 1, 2 und 3, die durch parallele oder simultane Ermittlungen der Marker gekennzeichnet ist.

5. Verfahren gemäß Anspruch 1 ist **dadurch gekennzeichnet, dass** die Bestimmungen durch die Verwendung eines automatischen Analysegeräts durchgeführt werden

## Revendications

1. Dosage immunologique de Procalcitonine pour le diagnostic in vitro pour un patient ayant un diabète sucré pancréatique et /ou du syndrome du pied diabétique, comprenant: la détermination du niveau de procalcitonine dans un échantillon choisi dans le groupe consistant en un échantillon de sang, un échantillon de sérum et un des échantillon de plasma obtenus à partir du sang du patient, avec un nombre de leucocytes dont la valeur normale varie de 4000 à 10 000 leucocytes par mm³ et la comparaison du niveau de procalcitonine par rapport au niveau seuil, par conséquent, lorsque le niveau de procalcitonine dépasse le niveau seuil en question, le patient est diagnostiqué avec un diabète sucré pancréatique et /ou du syndrome du pied diabétique, dont le niveau seuil est compris entre 0,03 ± 0.002ng /ml.

2. Procédé selon la revendication 1, dans lequel en outre au moins un marqueur inflammatoire est déterminée, qui est choisi dans le groupe de la protéine C-réactive (CRP), des cytokines telles que TNF-alpha, par exemple des inierleukines telles que IL-6, l'interleukine-1 β, proadrénomédulline (proADM), proadrénomédulline midregional (MR-proADM), l'angiotensine II ou de l'endothéline-1.

3. Procédé selon la revendication 1, dans lequel en outre au moins un marqueur vasculaire est déterminé, lequel est choisi dans le groupe de la créatine kinase, la myéloperoxydase, la myoglobine, protéine natriurétique, en particulier ANP (ou ANF), proANP, NT-proANP, BNP, proBNP, NT-proBNP, ou (pro) hormones qui régulent le système cardiovasculaire, tels que les pro-gastrin-releasing- peptide (proGRP), pro-endothéline-1, pro-leptine, pro-neuropeptide Y, pro-somatostatine, pro-neuropeptide -YY ou proopiomélanocortine.

4. Procédé selon les revendications 1, 2 et 3, **caractérisé en ce que** des déterminations parallèles ou simultanées des marqueurs sont effectuées.

5. Procédé selon la revendication 1, **caractérisé en ce que** les déterminations sont effectuées en utilisant un dispositif d'analyse automatisé.
